# EUROPÄISCHE PATENTSCHRIFT

(11) **EP 0 610 742 B1**
(45) Veröffentlichungstag und Bekanntmachung des Hinweises auf die Patenterteilung: **24.03.1999**
(21) Anmeldenummer: 94101257.7
(22) Anmeldetag: 28.01.1994
(51) Int. Cl.: C07D 311/72

(54) **Verfahren zur Gewinnung von Tocopherolen und Sterolen aus natürlichen Quellen**
Process for obtaining tocopherols and sterols from natural sources
Procédé d'obtention de tocophérols et de stérols à partir de sources naturelles

(30) Priorität: 11.02.1993 CH 467/93
(43) Veröffentlichungstag der Anmeldung: 17.08.1994
(73) Patentinhaber: F. HOFFMANN-LA ROCHE AG, 4002 Basel (CH)
(72) Erfinder: Fizet, Christian, F-68440 Zimmersheim (FR)
(74) Vertreter: Cottong, Norbert A.

(56) Entgegenhaltungen:
- US-A- 3 335 154

## Beschreibung

Die vorliegende Erfindung betrifft ein neues Verfahren zur Gewinnung von Tocopherolen und Sterolen aus natürlichen, insbesondere pflanzlichen Quellen.

Es ist bekannt, dass pflanzliche Oele und Fette, wie beispielsweise Sonnenblumenöl, Sojaöl, Rapsöl, Baumwollsaatöl, Palmöl, Erdnussöl, Weizenkeimöl und dergleichen, wertvolle natürliche Quellen von gewissen Tocopherolen und Sterolen darstellen. Solche Oele und Fette enthalten neben den erwähnten Tocopherolen und Sterolen auch u.a. freie Fettsäuren, Wachse und Glyzeride. Der Gewinnung der natürlichen Tocopherole aus diesen Quellen kommt grosse Bedeutung zu, was auch zunehmend für die Gewinnung der natürlichen Sterole gilt.

Es sind bereits vielerlei Verfahren zur Gewinnung von Tocopherolen und Sterolen aus pflanzlichen Oelen und Fetten bekannt. In diesen Verfahren eignen sich als unmittelbar zu verwendende Ausgangsmaterialien insbesondere die im Zusammenhang mit der Gewinnung von Speiseölen und -fetten nach der Desodorierungsstufe erhaltenen Destillate, die geläufig unter dem Namen "Brüdenabscheiderfette" bekannt sind. Hierfür verwendet man vorwiegend die Brüdenabscheiderfette von der Desodorierung von Sojaöl, wobei Brüdenabscheiderfette aus anderen pflanzlichen Oelen analog verwendet werden (können). Im allgemeinen handelt es sich bei den bekannten Techniken um die Ausnutzung der verhältnismässig niedrigen Löslichkeit kristalliner Sterole in Lösungsmitteln, wie niederen aliphatischen Alkoholen und Ketonen, gegenüber der Löslichkeit von Tocopherolen in solchen Lösungsmitteln. Aus der US-Patentschrift 2.866.797 ist ein Verfahren zur Isolierung von Sterolen aus pflanzlichen Quellen, insbesondere Oelen, bekannt, wobei man die Fettkomponente der pflanzlichen Quelle verseift, die wässrige Lösung der Verseifungsprodukte z.B. mit Dichloräthylen extrahiert, dem Extrakt Wasser und ein mit Wasser mischbares Lösungsmittel, z.B. Methanol oder Aethanol, zugibt und das resultierende Gemisch zur Ausfällung der Sterole abkühlt. Mehrere weitere US-Patentschriften, z.B. Nrn. 3.335.154, 3.122.565, 2.704.764 und 3.153.055, beschreiben Verfahren zur Isolierung von Tocopherolen und Sterolen aus Brüdenabscheiderfetten, wobei jeweils ein wesentlicher Verfahrensschritt darin besteht, das Brüdenabscheiderfett mit einem niederen aliphatischen Alkohol, z.B. Methanol, zu versetzen, und zwar normalerweise in Gegenwart eines sauren Katalysators. Gemäss der ersten der vier soeben erwähnten US-Patentschriften wird das Brüdenabscheiderfett allerdings vorher einer Verseifung, gefolgt von einer Ansäuerung, unterworfen. Bei der Behandlung mit Alkohol handelt es sich jeweils um die Veresterung der vorhandenen freien Fettsäuren, was u.a. bei erhöhter Temperatur (64-150°C, gemäss USP 2.704.764) erfolgen kann. Weitere typische Verfahren sind in Ullmanns Enzyklopädie der techn. Chemie 23, 643-649 und 685-692, Verlag Chemie, Weinheim 1983, und den US-Patentschriften 3.108.120 und 3.153.054 beschrieben. Im allgemeinen ist zu bemerken, dass gemäss dem Stand der Technik der Zusatz eines niederen Alkohols zur Veresterung der im Brüdenabscheiderfett vorhandenen Fettsäuren benötigt wird.

Die oben beschriebenen bekannten Prozeduren sind aufwendig. Es wurde nun gefunden, dass man die bereits in den jeweiligen Brüdenabscheiderfetten vorhandenen Fettsäuren und Sterole zur Esterbildung bringen kann, ohne dass die ebenfalls vorhandenen Tocopherole wesentlich miteinbezogen werden. Diese Tocopherole und die bei der Veresterung der Sterole mit den Fettsäuren gebildeten Ester (im folgenden "Sterolester" genannt) können dann anhand ihrer unterschiedlichen Destillierbarkeit voneinander getrennt und separat behandelt werden, um schliesslich zu den getrennten Tocopherolen und Sterolen zu gelangen. Dies stellt die Grundlage der vorliegenden Erfindung dar, die insbesondere ein Verfahren zum Abtrennen von Tocopherolen und Sterolen aus Brüdenabscheiderfetten durch Destillation dieser Brüdenabscheiderfette betrifft, welches Verfahren dadurch gekennzeichnet ist, dass man vor der Destillation die im Brüdenabscheiderfett vorhandenen Sterole mit den ebenfalls vorhandenen Fettsäuren verestert, das resultierende Gemisch zwecks Gewinnung von übrigbleibenden Fettsäuren und anschliessend zwecks Gewinnung von Tocopherolen destilliert, wobei die bei der Veresterung gebildeten Sterolester im Rückstand dieser Destillation zurückbleiben, und dann die Tocopherole aus dem Destillat und die Sterole nach Spaltung deren Ester aus dem Destillationsrückstand isoliert. Die Veresterung wird vorzugsweise durchgeführt, indem man das Brüdenabscheiderfett bei einer Temperatur von etwa 150°C bis etwa 250°C, wie beispielsweise von etwa 150°C bis etwa 220°C, während etwa 1 bis 12 Stunden erhitzt, wobei im allgemeinen höhere Temperaturen eine kürzere Erhitzungsdauer erfordern.

Wie oben angedeutet, kommen als Brüdenabscheiderfette insbesondere in Frage die Rückstände (Destillate) der Desodorierung der üblichen Speiseöle und Fette, wie z.B. Sonnenblumenöl-Destillate, Sojaöl-Destillate, Palmöl-Destillate usw. Es können jedoch auch zu diesem Zwecke die noch nicht desodorierten Oele und Fette selbst verwendet werden, sowie auch die erwähnten Rückstände, welche bereits einer weiteren Konzentrationsstufe unterworfen wurden. Die Bereitstellung solcher Brüdenabscheiderfette und die Apparatur zu diesem Zwecke sind in der Fachliteratur mehrmals beschrieben, z.B. im Artikel "Deodorization and Finished Oil Handling" von Arnold M. Gavin, J. Amer. Oil Chemists Soc. 58, 175-184, März 1981.

Diese Brüdenabscheiderfette enthalten in der Regel die Tocopherole sowie freie Fettsäuren, Glyzeride von denjenigen Säuren, die normalerweise in Oelen und Fette vorkommen, Salze von Fettsäuren, Sterole, Wachse, Squalen und einen Rest von z.T. nicht identifizierbaren Begleitstoffen. Der Ausdruck "Tocopherole" umfasst im Rahmen der vorliegenden Erfindung nicht nur die α-, β-, γ- und δ-Tocopherole, sondern auch die entsprechenden Tocopherol-Homologe, z.B. Tocotrienole, Tocodienole und Tocoenole, die in verhältnismässig kleinen Mengen vorliegen können. Der mengenmässige Anteil dieser Substanzen variiert in der Regel sehr stark je nach Art der Ausgangsmaterialien und deren Gewinnungsverfahren. In den vorhergehend erwähnten Desodorierungsrückständen (Destillaten) liegt der Anteil an Tocopherolen in der Regel zwischen etwa 1 und etwa 15 Gew.-% und derjenige von Sterolen zwischen etwa 4 und etwa 20 Gew.-%. Der Gesamtanteil an freien Fettsäuren, Glyzeriden und Salzen beträgt in der Regel von etwa 40 bis etwa 85 Gew.-% und derjenige des z.T. nicht identifizierbaren Restes von etwa 5 bis etwa 20 Gew.-%. In noch nicht desodorierten Fetten und Oelen, und in bereits weiter aufkonzentrierten Desodorierungsrückständen, liegen diese Werte bekanntlich entsprechend niedriger bzw. höher.

Durch das erfindungsgemässe Verfahren, das im wesentlichen aus einfachem Erhitzen des Brüdenabscheiderfettes besteht, werden die im Brüdenabscheiderfett vorhandene Sterole mit den ebenfalls vorhandenen Fettsäuren verestert. Dadurch lässt sich in der nachfolgenden Destillation bereits in einem sehr frühen Stadium des gesamten Verfahrens zum Abtrennen von Tocopherolen und Sterolen aus natürlichen, insbesondere pflanzlichen, Quellen ein grosser Teil der sonst störenden Sterole als Ester entfernen. Allerdings ist beim Erhitzen des Brüdenabscheiderfettes ein geringer Verlust an Tocopherolen nicht oder zumindest sehr schwer zu vermeiden, da die Tocopherole - wenngleich mit viel kleinerer Reaktionsgeschwindigkeit als derjenigen der Sterole - ebenfalls mit den Fettsäuren verestern (können).

Nicht nur die gebildeten Sterolester, sondern auch die im Brüdenabscheiderfett vorhandenen Glyzeride (Di- und Triglyzeride) und höher siedenden Bestandteile bleiben nach dem Erhitzen als Rückstand. Beispiele von Sterolen, die beim Erhitzen mit den Fettsäuren in die entsprechenden Sterolester übergeführt werden, sind hauptsächlich Campesterol, Stigmasterol und Sitosterol. Die eigentlichen Typen von Sterolen hängen selbstverständlich von der Natur (Herkunft, Herstellung usw.) des jeweils verwendeten Brüdenabscheiderfetts ab.

Verschiedene sonstige Reaktionsbedingungen können den Ablauf und das Ergebnis des Erhitzens beeinflussen. So verhalten sich während des Erhitzens die verschiedenen im Brüdenabscheidefett vorhandenen Tocopherole nicht absolut gleich, d.h. gewisse Tocopherole pflegen etwas mehr (und natürlich unerwünscht) in der Veresterung miteinbezogen zu werden als andere, was zu einem entsprechend unterschiedlichen Verlust an Tocopherolen führt. Bei α-Tocopherol (z.B. in aus Sonnenblumenöl hergestelltem Brüdenabscheiderfett) ist beispielsweise der Verlust äusserst gering, während der Gehalt an γ und δ-Tocopherolen unter sonst gleichen Veresterungsbedingungen stärker abnehmen kann, was bei der Veresterung von aus Sojaöl hergestelltem Brüdenabscheiderfett eine grössere Rolle spielt. In diesen Fällen wird hingegen die Esterbildung aus den vorhandenen Fettsäuren und Sterolen nicht oder kaum beeinflusst. Der Verlust an Tocopherolen kann durch vorhergehende Entgasung des Brüdenabscheiderfettes wesentlich reduziert werden, z.B. um ca. die Hälfte. Im allgemeinen stellt in allen Fällen eine solche vorhergehende Entgasung ein bevorzugtes Merkmal des erfindungsgemässen Verfahrens dar. Eine typische Entgasung erfolgt bei 60-70°C bei einem Druck von ca. 20 mbar und für die Dauer von 15-20 Minuten. Des weiteren kann das Verfahren bei normalem oder erhöhtem Druck erfolgen, und zwar mit dem Ergebnis, dass die Reaktionsgeschwindigkeit (der Veresterung) je nach Druck leicht beeinflusst wird. Aus wirtschaftlichen Gründen führt man das erfindungsgemässe Verfahren vorzugsweise bei normalem Druck durch. Die Zugabe einer starken Säure als Katalysator beschleunigt stark die Veresterung, was allerdings auch für die unerwünschte Veresterung der Tocopherole gilt. Demzufolge ist es unnötig, das erfindungsgemässe Verfahren, etwa durch Zugabe einer Säure zum bereits schwach sauren (pH 5-6) Brüdenabscheiderfett, zu katalysieren. Die Zugabe von Basen, z.B. Natriumhydroxid, die sofort von den vorhandenen Fettsäuren neutralisiert werden, beeinflusst die Esterbildung nicht, erniedrigt jedoch ein wenig die Ausbeute an Tocopherolen, so dass auch die Zugabe von Basen nicht von Vorteil, sondern womöglichst zu vermeiden ist. Was die Reaktionstemperatur anbelangt, so hat es sich erwiesen, dass im allgemeinen höhere Temperaturen zu kürzeren Reaktionszeiten führen. Aus praktischen und wirtschaftlichen Gründen erfolgt die im Kernpunkt des erfindungsgemässen Verfahrens stehende Veresterung vorzugsweise im Temperaturbereich von etwa 150°C bis etwa 250°C, und zwar während etwa 1 bis 12 Stunden. Ganz bevorzugt wird die Veresterung im Bereich von etwa 180°C/2,5 Stunden bis etwa 250°C/1,5 Stunden durchgeführt.

Nach der Veresterung des Brüdenabscheiderfetts durch Erhitzen können die übrigbleibenden Fettsäuren sowie weitere leichtflüchtige Begleitstoffe, die hauptsächlich pro Molekül bis 20 Kohlenstoffatome aufweisen, abdestilliert werden, und zwar ohne allzugrossen Verlust an den gewünschten Tocopherolen. Dadurch wird die Konzentration der Tocopherole in der zurückbleibenden Flüssigkeit weiter erhöht. Das erhaltene Fettsäuredestillat, das als die "erste Destillationsfraktion" bezeichnet werden kann, enthält typischerweise ca. 70 bis 90 Gewichtsprozent Fettsäuren und kann als solches verwertet werden; d.h. die Fettsäuren können nach Wunsch zurückgewonnen werden. Diese erste Destillation, welche die erste Destillationsfraktion ergibt, erfolgt zweckmässigerweise bei ca. 0,1 mbar Druck und bei Heizbad-(z.B. Oelbad)Temperaturen im Bereich von ca. 120 bis ca. 150°C. Als Destillationsapparatur kann man z.B. einen Kurzwegverdampfer verwenden. Die Destillationsbedingungen können selbstverständlich je nach Inhalt der Apparatur und Typ und Grösse der Apparatur verschieden sein.

Nach weiterer Destillation erhält man eine Destillationsfraktion (die "zweite Destillationsfraktion"), die gegenüber dem veresterten Brüdenabscheiderfett einen viel höheren Gehalt an Tocopherolen aufweist, und zwar typischerweise ca. 15 bis ca. 40 Gewichtsprozent davon. Die bei der Veresterung gebildeten Sterolester bleiben im Rückstand der gesamten Destillation, während allfällige unveresterte Sterole zusammen mit dem Grossteil der ursprünglich vorhandenen Tocopherole sich in der zweiten Destillationsfraktion befinden. Diese restlichen Sterole stören nicht und können mit anderen Begleitstoffen während der weiteren Verarbeitung der zweiten Destillationsfraktion abgetrennt werden. Die zweite Destillation, welche die zweite Destillationsfraktion ergibt, wird zweckmässigerweise bei Heizbad-(z.B. Oelbad)Temperaturen im Bereich von ca. 200 bis ca. 220°C durchgeführt. Die zweckmässigen Destillationsbedingungen sind ansonsten analog den im Zusammenhang mit der ersten Destillation oben angegebenen Bedingungen.

Die in der zweiten Destillationsfraktion vorhandenen verbliebenen sauren Komponenten, hauptsächlich Fettsäuren, sowie Squalen können nach verschiedenen Methoden entfernt werden, um eine noch stärkere Anreicherung der Tocopherole im Destillat zu erreichen. Eine solche Methode besteht darin, die sauren Komponenten in ihre entsprechenden Ester, vorzugsweise Methylester, umzuwandeln. Die in Methanol durchgeführte Veresterung verläuft nahezu quantitativ und ohne wesentlichen Verlust der ebenfalls in der zweiten Destillationsfraktion vorhandenen Tocopherole. Zweckmässigerweise wird die Veresterung durch Spuren von zugegebenen Säuren, insbesondere einer Mineralsäure, wie beispielsweise Salzsäure, katalysiert und im Temperaturbereich von ca. 65°C bis ca. 100°C durchgeführt. Eine typische Veresterung mit Methanol verläuft bei ca. 65°C in Gegenwart von Salzsäure als Katalysator, und zwar ca. 5 Stunden lang. Eine weitere Methode zum Entfernen der sauren Komponenten und zugleich zum Anreichern der Tocopherole im Destillat besteht darin, dass man das Destillat mit Calciumhydroxid in Gegenwart von Wasser in einem inerten, mit Wasser mischbaren organischen Lösungsmittel, insbesondere einem niederen Alkanol, wie beispielsweise Isopropanol, behandelt und anschliessend die so gebildeten Calciumsalze abtrennt. Dadurch, dass diese Salzbildung in Gegenwart von Wasser durchgeführt wird, werden zudem allfällig noch vorhandene Glyzeride unter Bildung von Fettsäure-Calciumsalzen verseift. Die Abtrennung der so erhaltenen Calciumsalze erfolgt zweckmässigerweise durch Entfernen, z.B. Abdestillieren unter vermindertem Druck, des verwendeten organischen Lösungsmittels und Ausfällung aus einem geeigneten weiteren Lösungsmittel, z.B. einem Niederalkylformiat oder -acetat, einem leicht flüchtigen Keton oder einem leicht flüchtigen Nitril. Diese zweite Methode, die allerdings zum Anreichern von Tocopherolen in den oben beschriebenen Brüdenabscheiderfetten vorgesehen ist aber ebenfalls für deren Anreichern in der nun in Frage stehenden zweiten Destillationsfraktion eingesetzt werden kann, ist in der Europäischen Patentschrift Nr. 316 729 näher beschrieben und exemplifiziert.

Das nach der oben beschriebenen Verarbeitung der zweiten Destillationsfraktion (Entfernung der sauren Komponenten) resultierende Konzentrat kann einer Ionenaustauschprozedur unterworfen werden, um die erwünschten Tocopherole, insbesondere über ein stark basisches Harz, selektiv zu absorbieren. Als Phenole zeigen die Tocopherole eine gewisse, wenn auch sehr schwache, Acidität. Deswegen können sie mit verschiedenen Arten stark basischer Ionenaustauscher Salze bilden, und zwar sogenannte "Tocopherolate". Zu diesem Zwecke kann man insbesondere im Handel erhältliche Ionenaustauscher, wie beispielsweise Amberlite® IRA 900, Amberlyst® A-26, Duolite® A-161, Levatit® 500 MB, Dowex®1x2 und XUS® 40240, verwenden, von denen Amberlite® IRA 900 bevorzugt ist. Als Eluierungsmittel verwendet man zweckmässigerweise u.a. ein niederes Alkanol, z.B. Methanol, Aethanol oder Isopropanol, oder ein aliphatisches Keton, z.B. Aceton. Auf diese Weise werden die meisten, wenn nicht alle, Begleitstoffe eluiert und infolgedessen von den Tocopherolen abgetrennt. Letztere können dann beispielsweise mit Essigsäure/Isopropanol-Mischungen desorbiert werden, und zur Regenerierung des Harzes kann man geeigneterweise Kali- oder Natronlauge, insbesondere deren 4-prozentige Lösungen, verwenden. Das nach dieser Ionenaustauschprozedur resultierende Tocopherolkonzentrat weist eine sehr hohe Reinheit auf, oftmals über 93%. Es stellt sich heraus, dass die identifizierbaren Begleitstoffe, die lediglich in Spuren vorhanden sind, Tocopherol-Homologe (Vorläufer) sind, wie beispielsweise δ-Tocoenol, γ-Tocoenol, γ-Tocodienol, γ-Tocotrienol, α-Tocoenol und dergleichen, die gleichfalls Vitamin E-Wirkung besitzen, so dass sie nicht als "Verunreinigungen" betrachtet werden müssen. Somit liegt der infolge der Ionenaustauschprozedur erreichte Gehalt an Tocopherolen oftmals sogar über 95%.

Das nach der Ionenaustauschprozedur erhaltene Konzentrat kann schliesslich weiter gereinigt werden, indem man es einer Flach- oder Kugelrohrdestillation unterwirft. Dadurch werden das restliche Lösungsmittel sowie Spuren an schwerflüchtigen Komponenten (Polymeren, Wachsen usw.) abgetrennt, wobei nur ein geringer Verlust an Tocopherolen, oftmals nicht mehr als 2-3 Gewichtsprozent, zu verzeichnen ist. Auf diese Weise kann ein Konzentrat, das beispielsweise einen ca. 94%igen Gehalt an Tocopherolen aufweist, in ein Konzentrat mit einem ca. 97%igen Gehalt an Tocopherolen umgewandelt werden. Die Destillation wird zweckmässigerweise analog der oben beschriebenen ersten und zweiten Destillation durchgeführt, wobei allerdings die zweckmässigen Heizbad-(z.B. Oelbad-)Temperaturen im Bereich von ca. 240 bis ca. 260°C liegen und ein Kugelrohrofen als bevorzugte Destillationsapparatur verwendet wird. Das Produkt dieser Destillation stellt das Endprodukt des gesamten Verfahrens zur Gewinnung von Tocopherolen aus natürlichen Quellen dar.

Der nach dem Erhitzen (der Veresterung) des Brüdenabscheiderfettes und der anschliessenden Destillation erhaltene Rückstand enthält den Hauptteil der in der Veresterung gebildeten Sterolester, aus denen die Sterole selber gewonnen werden können. Neben diesen schwerflüchtigen, wachsartigen Sterolestern befinden sich im Rückstand Triglyzeride, weitere Wachse sowie zahlreiche hochmolekulare Begleitstoffe teils unbekannter Natur. Bei der Sterolgewinnung handelt es um eine säurekatalysierte Umesterung der Fettsäure-Sterol-Ester und von verhältnismässig geringen Mengen an Fettsäure-Tocopherol-Estern mit einem niederen Alkanol, insbesondere Methanol oder Aethanol, zu den entsprechenden Sterolen, Tocopherolen und Fettsäurealkylestern. Die Umesterung wird zweckmässigerweise in einem Stahlautoklaven durchgeführt. Als sauren Katalysator verwendet man vorzugsweise konzentrierte Schwefelsäure, und als Lösungsmittel das überschüssige Alkanol. Zudem erfolgt die Umesterung zweckmässigerweise bei Temperaturen zwischen ca. 70 und ca. 150°C, und zwar innert einer Dauer von ca. 1 bis ca. 5 Stunden. Durch die Wahl geeigneter Katalysatoren, Lösungsmittel, Reaktionsdauer und Temperatur kann eine Optimierung der Ausbeute an Sterolen erreicht werden. Die schwer zu vermeidenden Hauptverunreinigungen bestehen aus dehydratisierten Sterolen, wobei man allerdings in der Regel eine gute Ausbeute an den gewünschten Sterolen erreicht, sowohl in bezug auf den Gehalt der Sterole im ursprünglichen Brüdenabscheiderfett als auch in bezug auf den Sterolgehalt im Ausgangsmaterial (Rückstand). Zur vollständigen Abtrennung der Sterole wird die Reaktionslösung abgekühlt, zweckmässigerweise auf ca. 0°C. Durch Filtration dieser Lösung, die infolge der Kühlung Kristalle von Sterolen enthält, können die Sterole von der Mutterlauge getrennt werden. Die im Filtrat verbliebenen Sterole und Tocopherole können recyclisiert werden, so dass die Ausbeute an diesen in der ganzen Prozedur auf diese Weise noch erhöht werden kann.

Die vorliegende Erfindung wird anhand der nachfolgenden Beispiele veranschaulicht.

### Beispiel 1

Als Ausgangsmaterial (Brüdenabscheiderfett) wird ein Sonnenblumenöl-Destillat folgender Zusammensetzung verwendet:

| | Gew.-% |
|---|---|
| Tocopherole | 3,5 |
| Sterole | 12,5 |
| Fettsäuren | 34 |
| Glyzeride | 20 |
| Squalen | 2,4 |
| Rest | 27,6 |

In einem Rollverdampfer werden 744 g des obigen Brüdenabscheiderfetts bei 170°C und Wasserstrahlvakuum 9 Stunden erhitzt. Nach dieser Veresterungsdauer erhält man 721 g "verestertes" Brüdenabscheiderfett.

716 g des veresterten Brüdenabscheiderfetts werden in einem Kurzwegverdampfer (0,025 m²), versehen mit einem Doppelmanteldosiertrichter und einem Nadelventil, einer Destillation bei 0,1 mbar und bei einer Oeltemperatur von 133°C unterworfen. Danach erhält man 275,6 g Fettsäuredestillat sowie 428,5 g Rückstand. Nach anschliessender Destillation von ca. 425 g dieses Rückstandes bei 0,1 mbar und 207°C erhält man 128,2 g eines Konzentrats sowie 296,1 g Rückstand. Es wird etabliert, dass das Konzentrat einen 19,4%-igen Gehalt an Tocopherolen aufweist, wobei die Ausbeute (bezogen auf die im Ausgangsmaterial vorhandene Menge an Tocopherolen) an Tocopherolen mehr als 95% beträgt.

Die ganze obige Prozedur wird mit dem Unterschied wiederholt, dass man die Veresterung des Brüdenabscheiderfetts 4 Stunden bei 200°C anstatt 9 Stunden bei 170°C durchführt. Die Ergebnisse sind ähnlich.

### Beispiel 2

Als Ausgangsmaterial (Brüdenabscheiderfett) wird ein Sojaöl-Destillat folgender Zusammensetzung verwendet:

| | Gew.-% |
|---|---|
| Tocopherole | 10,2 |
| Sterole | 11,2 |
| Fettsäuren und Ester | 51,8 |
| Squalen | 1,9 |
| Rest (Glyzeride und andere hochmolekulare Substanzen) | 24,9 |

Vier Veresterungen werden mit gleichen Teilen des obigen Brüdenabscheiderfetts durchgeführt, und zwar unter verschiedenen Bedingungen hinsichtlich der Veresterungsdauer. Diese besteht aus einer Aufheizperiode (140°C→200°C) und einer Heizperiode bei konstanter Temperatur (200°C).

Man führt die Veresterungen jeweils in einem 63 l-Edelstahlrührkessel mit Mantelheizung und Oelthermostat durch. Nach Entgasen während 1,5 Stunden bei 60°C/15 mmHg wird das jeweilige Brüdenabscheiderfett schliesslich auf 200°C erhitzt und bei dieser Temperatur gehalten. Anschliessend wird die Zusammensetzung jedes Veresterungsproduktes gas-chromatographisch ermittelt. Die Ergebnisse sind in der nachfolgenden Tabelle 1 angegeben:

**Tabelle 1**

| **Versuch** | **Edukt** | **1** | **2** | **3** | **4** |
|---|---|---|---|---|---|
| Aufheizperiode (140→200°C; h) | | 0,7 | 1,2 | 1,2 | 2,0 |
| | | | | | |
| Heizperiode (200°C; h) | | 3,5 | 2,4 | 1,7 | 1,0 |
| | | | | | |
| Tocopherole (Gew.-%) | 10,2 | 8,7 | 8,9 | 9,4 | 9,4 |
| | | | | | |
| Sterole (Gew.-%) | 11,2 | 0,6 | 1,0 | 1,7 | 2,1 |
| | | | | | |
| Fettsäuren und Ester (Gew.-%) | 51,8 | 33,8 | 33,9 | 39,5 | 40,7 |
| | | | | | |
| Veresterte Tocopherole (Gew.-%) | | 14,0 | 12,1 | 7,9 | 7,4 |

Vor der anschliessenden Destillation werden die veresterten Brüdenabscheiderfette aus den vier Veresterungsversuchen wieder vereint. Das vereinigte Material weist gemäss Gaschromatographie folgende Zusammensetzung auf:

| | Gew.-% |
|---|---|
| Tocopherole | 9,3 |
| Sterole | 1,6 |
| Fettsäuren und Ester | 39,4 |
| Squalen | 1,8 |
| Rest | 46,6 |

Die Angaben zeigen, dass der Tocopherolgehalt leicht zurückgegangen ist (9,3 Gew.-% gegenüber 10,2 Gew.-%), während der Gehalt an Sterolen sich stark verringert hat (1,6 Gew.-% gegenüber 11,2 Gew.-%). Die infolge des Erhitzens gebildeten Sterolester befinden sich nun im "Rest"-Material.

Nach der Veresterung wird eine zweistufe Kurzwegdestillation von Portionen des vereinigten veresterten Brüdenabscheidefettes durchgeführt, und zwar in einer zwei Kurzwegverdampfer (im folgenden als "KWV 1" bzw. "KWV 2" bezeichnet) aufweisenden Destillationsapparatur. Zur Ermittlung der optimalen Betriebsbedingungen werden sowohl der Druck ("D") als auch die Temperatur ("T") in beiden Kurzwegverdampfern variiert. In der nachfolgenden Tabelle 2 sind die zur Optimierung der Destillationsbedingungen wesentlichen Betriebsparameter und die zur Beurteilung entscheidenden Konzentrationen der Fettsäuren ("Fs") und Tocopherole ("Toc") in den einzelnen Fraktionen [Fettsäurefraktion ("FsF") und Tocopherolfraktion ("TocF")] zusammengefasst:

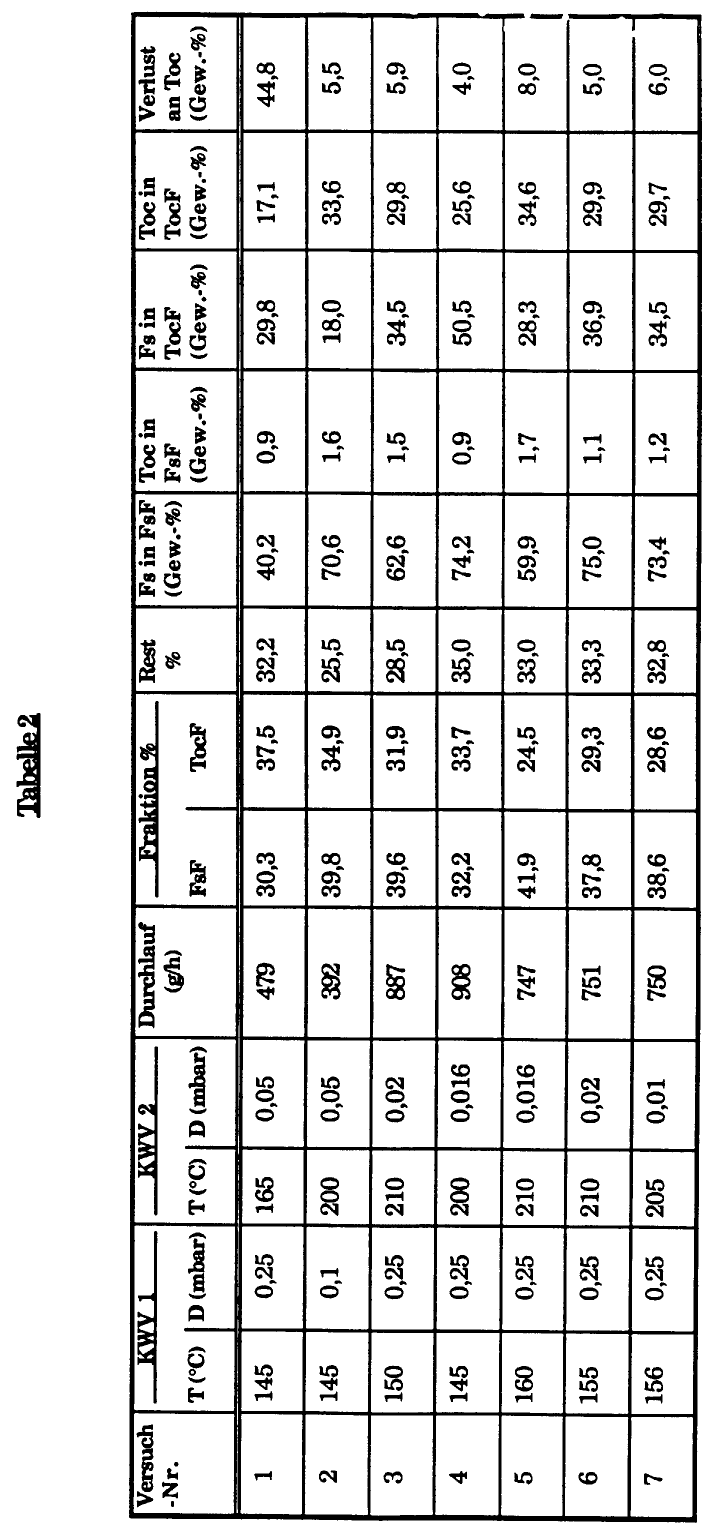

Die Angaben zeigen, dass die Tocopherolfraktionen einen gegenüber dem veresterten Brüdenabscheider (Ausgangsmaterial der Destillation) viel höheren Gehalt an Tocopherolen und in fast allen Fällen einen niedrigeren Gehalt an Fettsäuren aufweisen. Ihrerseits weisen die Fettsäurefraktionen einen gegenüber dem veresterten Brüdenabscheiderfett viel höheren Gehalt an Fettsäuren und einen wesentlich niederen Gehalt an Tocopherolen auf. Die Sterolester befinden sich im "Rest"-Material. Der Verlust an Tocopherolen ist im allgemeinen gering.

### Beispiel 3

Als Ausgangsmaterial (eine nach der in Beispiel 2 beschriebenen zweistufigen Kurzwegdestillation erhaltene Tocopherolfraktion, d.h. eine "zweite" Destillationsfraktion) in der nachfolgend beschriebenen Fettsäure-Veresterung wird eine Destillationsfraktion folgender Zusammensetzung verwendet:

| | Gew.-% |
|---|---|
| Tocopherole | 31 |
| Sterole | 5,1 |
| Fettsäuren | 32 |
| Squalen | 4,9 |

99,6 g der Tocopherolfraktion werden in 150 ml Methanol gelöst. Zu der Lösung werden 5 Tropfen (ca. 100 mg) 36%ige Salzsäure gegeben und das Reaktionsgemisch wird 5 Stunden unter Rühren und Argonbegasung bei Rückflusstemperatur erhitzt. Danach wird das Gemisch auf Raumtemperatur abgekühlt und unter vermindertem Druck das Lösungsmittel abgedampft. Man erhält auf diese Weise 101,8 g eines Konzentrats, das gemäss Gaschromatographie einen 30,3%igen Gehalt an Tocopherolen (ca. 100% Ausbeute) aufweist.

### Beispiel 4

Als Ausgangsmaterial wird in der nachfolgend beschriebenen Ionenaustauschprozedur das Konzentrat verwendet, dessen Bereitstellung in Beispiel 3 beschrieben ist. Man verwendet in dieser Prozedur eine mit 150 g Amberlite® IRA 900 (20-50 mesh) gefüllte Glassäule vom Durchmesser 5 cm. Das Konzentrat weist anfangs folgende Zusammensetzung auf:

| | Gew.-% |
|---|---|
| Tocopherole | 30,3 |
| Sterole | 5,8 |
| Squalen | 4,6 |

Das Harz (Amberlite® IRA 900, 20-50 mesh; Acetat-Form) wird mit 200 ml entionisiertem Wasser gewaschen und anschliessend mit 1 l 4%igem wässrigem Kaliumhydroxid aktiviert. Die überschüssige Kaliumhydroxidlösung wird mit 300 ml entionisiertem Wasser herausgewaschen und seinerseits das Wasser mit 600 ml Methanol verdrängt. Man löst 101,3 g obigen Konzentrats in 250 ml Isopropanol und gibt die resultierende Lösung auf das Harz. Dann wird die Säule mit 400 ml Isopropanol eluiert, um die Begleitstoffe abzutrennen. Anschliessend gibt man 200 ml eines Methanol/Essigsäure(4:1)-Gemisches auf das Harz und lässt das so befeuchtete Harz ca. 3 Stunden stehen, wobei der Hahn geschlossen bleibt. Mit 600 ml Methanol werden danach die Tocopherole vollständig eluiert, und die resultierende saure, Tocopherol-haltige Methanol-Lösung (ca. 800 ml) wird dann unter vermindertem Druck eingeengt. Die so erhaltende Rohware (ca. 94%iger Tocopherolgehalt gemäss Gaschromatographie) wird in einem Kugelrohrofen (GKR 50) destilliert, und zwar bei 250°C und 0,1 mbar. Auf diese Weise erhält man 29,5 g eines leicht gelblichen Oels mit einem ca. 96%igen Gehalt an Tocopherolen (gemäss Gaschromatographie; 92% Ausbeute).

### Beispiel 5

Analog der in Beispiel 4 beschriebenen Prozedur erhält man aus 50,0 g eines Konzentrats, das einen 63,1%igen Gehalt an Tocopherolen, einen 9,5%igen Gehalt an Sterolen und Triterpenen und einen 8,6%igen Gehalt an Squalen aufweist, 30,6 g eines leicht gelblichen Oels. Dieses Oel enthält ca. 96,5 Gewichtsprozent an Tocopherolen (93,6% Ausbeute).

### Beispiel 6

Als Ausgangsmaterial wird in der nachfolgend beschriebenen Ionenaustauschprozedur das gleiche Konzentrat, welches in Beispiel 5 beschrieben ist, verwendet. Die Glassäule vom Durchmesser 5 cm wird mit 180 g Amberlite® IRA 900 (20-50 mesh) gefüllt. Dieses Harz wird mit 200 ml entionisiertem Wasser gewaschen und anschliessend mit 1 l 4%iger wässriger Kaliumhydroxidlösung reaktiviert. Die überschüssige Kaliumhydroxidlösung wird mit 300 ml entionisiertem Wasser herausgewaschen und seinerseits bei 0,1 bar Inertgas-Ueberdruck herausgepresst. Das restliche Wasser verdrängt man mit 300 ml Isopropanol. Man löst 40,3 g obigen Konzentrats in 230 ml Isopropanol und gibt die resultierende Lösung auf das Harz. Dann wird die Säule mit 400 ml Isopropanol eluiert, um die Begleitstoffe abzutrennen. Anschliessend gibt man 200 ml eines Isopropanol/Essigsäure(4:1)-Gemisches auf das Harz und lässt das so befeuchtete Harz ca. 3 Stunden stehen, wobei der Hahn geschlossen bleibt. Mit weiteren 400 ml des Isopropanol/Essigsäure-Gemisches werden danach die Tocopherole vollständig eluiert, das restliche Gemisch wird mit einem Inertgas-Ueberdruck von 0,1 bar herausgepresst, und die resultierende saure, Tocopherol-haltige Methanol-Lösung (ca. 600 ml) wird dann unter vermindertem Druck eingeengt. Die so erhaltene Rohware wird in einem Kugelrohrofen (GKR 50) destilliert, und zwar bei 250°C und 0,1 mbar. Auf diese Weise erhält man 24,3 g eines leicht gelblichen Oels mit einem ca. 96,5%igen Gehalt an Tocopherolen (gemäss Gaschromatographie; 92,3% Ausbeute).

### Beispiel 7

Bei dem in diesem Beispiel beschriebenen Sterolgewinnungsverfahren wird als Ausgangsmaterial ein Rückstand verwendet, der aus der Veresterung eines Sojaöl-Brüdenabscheiderfettes (wie diese beispielsweise in Beispiel 1 oder 2 beschrieben ist) resultiert. 15 g eines solchen Rückstands, enthaltend 4,9 g Sterole als Sterolester, 150 ml Methanol sowie 1,5 g konzentrierte Schwefelsäure, werden in einem mit Thermometer und Magnetrührer versehenen 250 ml-Stahlautoklaven vorgelegt. Dieser wird dann in ein auf 95°C vorgewärmtes Oelbad eingetaucht und der Inhalt des Autoklavs 3 Stunden bei dieser Temperatur gerührt. Nach Abkühlung der Lösung auf Raumtemperatur wird der Autoklav entleert und die Lösung in einem Rundkolben zur vollständigen Kristallisation der in der Reaktion gebildeten Sterole 1 Stunde bei 0°C belassen. Anschliessend werden die Sterole abfiltriert, mit wenig kaltem Methanol gewaschen und 1 Stunde bei 60°C unter Hochvakuum getrocknet. Man erhält auf diese Weise 4,9 g beiges Pulver. Gemäss Gaschromatographie beträgt die Ausbeute an Sterolen 90% bezogen auf das ursprüngliche Brüdenabscheiderfett bzw. nahezu 100% bezogen auf das Ausgangsmaterial (den Rückstand der Brüdenabscheiderfett-Veresterung).

Die oben beschriebene Prozedur erzielt unter Variieren der Reaktionszeit und der Innentemperatur (des Reaktionsgemisches) verschiedene Ergebnisse in bezug auf die Ausbeute an Sterolen und die Qualität des Endproduktes (prozentualer Gehalt an Sterolen im Produkt). In der nachfolgenden Tabelle 3 sind die Ergebnisse von 5 Versuchen unter Verwendung des gleichen Rückstandes wie oben (Gehalt an Sterolen 32,5 Gew.-%) angegeben:

**Tabelle 3**

| **Versuch** | **Reaktionszeit (Stunden)** | **Innentemp. (°C)** | **Ausbeute an Sterolen** | **Qualität des Endproduktes** |
|---|---|---|---|---|
| 1 | 5 | 105 | 57% | 63% Sterole (+ 24% dehydratisierte Sterole) |
| 2 | 2 | 105 | 75% | 83% Sterole |
| 3 | 1 | 100 | 80% | 88% Sterole |
| 4 | 2 | 85 | 76% | 70% Sterole |
| 5 | 3 | 85 | 90% | 90% Sterole |

## Patentansprüche

1. Verfahren zum Abtrennen von Tocopherolen und Sterolen aus Brüdenabscheiderfetten durch Destillation dieser Brüdenabscheiderfette, dadurch gekennzeichnet, dass man vor der Destillation die im Brüdenabscheiderfett vorhandenen Sterole mit den ebenfalls vorhandenen Fettsäuren verestert, das resultierende Gemisch zwecks Gewinnung von übrigbleibenden Fettsäuren und anschliessend zwecks Gewinnung von Tocopherolen destilliert, wobei die bei der Veresterung gebildeten Sterolester im Rückstand dieser Destillation zurückbleiben, und dann die Tocopherole aus dem Destillat und die Sterole nach Spaltung deren Ester aus dem Destillationsrückstand isoliert.

2. Verfahren nach Anspruch 1, dadurch gekennzeichnet, dass man zur Veresterung das Brüdenabscheiderfett bei einer Temperatur von etwa 150°C bis etwa 250°C während etwa 1 bis 12 Stunden erhitzt.

3. Verfahren nach Anspruch 2, dadurch gekennzeichnet, dass man bei einer Temperatur von etwa 150°C bis etwa 220°C erhitzt.

4. Verfahren nach Anspruch 2, dadurch gekennzeichnet, dass man bei etwa 180°C/2,5 Stunden bis etwa 250°C/1,5 Stunden erhitzt.

5. Verfahren nach einem der Ansprüche 1 bis 4, dadurch gekennzeichnet, dass man als Brüdenabscheiderfett ein Destillat der Desodorierung von Sonnenblumenöl, Sojaöl oder Palmöl verwendet.

6. Verfahren nach einem der Ansprüche 1 bis 5, dadurch gekennzeichnet, dass man vor der Veresterung das Brüdenabscheiderfett entgast.

7. Verfahren nach einem der Ansprüche 1 bis 6, dadurch gekennzeichnet, dass man die Veresterung bei normalem Druck durchführt.

## Claims

1. A process for the separation of tocopherols and sterols from deodorizer sludges by the distillation of this deodorizer sludge, characterized by, prior to the distillation, esterifying the sterols present in the deodorizer sludge with the fatty acids which are also present, distilling the resulting mixture to obtain residual fatty acids and subsequently to obtain tocopherols, whereby the sterol esters formed in the esterification remain in the residue of this distillation, and then isolating the tocopherols from the distillate and isolating the sterols, after cleavage of their esters, from the distillation residue.

2. A process according to claim 1, characterized in that the deodorizer sludge is esterified by heating at a temperature of about 150°C to about 250°C for about 1 to 12 hours.

3. A process according to claim 2, characterized in that the heating is carried out at a temperature of about 150°C to about 220°C.

4. A process according to claim 2, characterized in that the heating is carried out at about 180°C/2.5 hours to about 250°C/1.5 hours.

5. A process according to any one of claims 1 to 4, characterized in that a distillate from the deodorization of sunflower oil, soya oil or palm oil is used as the deodorizer sludge.

6. A process according to any one of claims 1 to 5, characterized in that the deodorizer sludge is de-gassed prior to the esterification.

7. A process according to any one of claims 1 to 6, characterized in that the esterification is carried out at normal pressure.

## Revendications

1. Procédé de séparation de tocophérols et de stérols, provenant de matières grasses de condenseurs de vapeurs, par distillation de ces matières grasses de condenseurs de vapeurs, caractérisé en ce que l'on estérifie avant la distillation les stérols présents dans la matière grasse des condenseurs de vapeurs avec les acides gras également présents, en ce qu'on distille le mélange résultant pour obtenir les acides gras restants et ensuite pour obtenir les tocophérols, dans lequel les esters de stérol formés lors de l'estérification restent dans le résidu de cette distillation, et on isole ensuite les tocophérols du distillat et les stérols du résidu de distillation après clivage de leurs esters.

2. Procédé selon la revendication 1, caractérisé en ce qu'on chauffe la matière grasse de condenseur de vapeurs pour l'estérifier à une température d'environ 150°C jusqu'à environ 250°C pendant environ 1 à 12 heures.

3. Procédé selon la revendication 2, caractérisé en ce qu'on chauffe à une température d'environ 150°C jusqu'à environ 220°C.

4. Procédé selon la revendication 2, caractérisé en ce qu'on chauffe à environ 180°C/2,5 heures jusqu'à environ 250°C/1,5 heures.

5. Procédé selon l'une des revendications 1 à 4, caractérisé en ce qu'on utilise comme matière grasse de condenseur de vapeurs un distillat de la désodorisation d'huile de tournesol, d'huile de soja ou d'huile de palme.

6. Procédé selon l'une des revendications 1 à 5, caractérisé en ce qu'on dégaze la matière grasse de condenseur de vapeurs avant l'estérification.

7. Procédé selon l'une des revendications 1 à 6, caractérisé en ce qu'on réalise l'estérification à pression normale.
